# EUROPEAN PATENT APPLICATION

(11) **EP 4 557 254 A1**
(43) Date of publication of application: **21.05.2025**
(21) Application number: 23209731.1
(22) Date of filing: 14.11.2023
(51) Int. Cl.: G08B 21/04, A61B 5/00

(54) **ARRANGEMENT FOR MONITORING A FIRST USER**

(71) Applicant: FireCat Group d. o. o., 3000 Celje (SI)
(72) Inventor: HOLMQVIST, Anders, Värmdö (SE); MAROLT, Bostjan, Bostanj (SI); ODER, Andreja, Mislinja (SI)
(74) Representative: Kransell & Wennborg KB

(57) **Abstract**

The invention concerns an arrangement for monitoring a first user at a first location, the arrangement comprising a sensor module (14) including at least one body sensor (38) and a garment (10) to be worn by the first user, the garment comprising a sensor module holder (12) for the sensor module (14), the sensor module holder (12) being located in and fastened to a first part (P1) of the garment (10) that is to press the sensor module holder (12) with the sensor module (14) against a part of a body of the first user when the first user wears the garment (10).

## Description

### TECHNICAL FIELD OF THE INVENTION

The present invention relates to an arrangement for monitoring a first user.

### DESCRIPTION OF RELATED ART

Workers in emergency services, like rescue workers such as firemen or policemen, often need to enter dangerous or hazardous environments. In these situations, it may be of interest to monitor the health of the workers in order to ensure that they are kept as safe as possible.

Today there exist various ways in which health can be monitored. For instance, there do exist applications in smart watches that monitor the pulse of a user that wears the smart watch.

However, these watches are not designed for the harsh and dangerous environment in which emergency service workers operate. Furthermore, such a worker is typically occupied and has no time or interest to operate the application. Therefore, it is of interest if the health monitoring is made with a minimum of involvement of the user.

Aspects of the present disclosure address situations such as these.

### SUMMARY OF THE INVENTION

The present invention is therefore directed towards monitoring the health of users located in dangerous and hazardous locations.

This object is according to a first aspect achieved through an arrangement for monitoring a first user at a first location, where the arrangement comprises:
a sensor module including at least one body sensor, and
a garment to be worn by the first user, the garment comprising a sensor module holder for the sensor module, the sensor module holder being located in and fastened to a first part of the garment that is to press the sensor module holder with the sensor module against a part of a body of the first user when the first user wears the garment.

The first part of the garment may be at least partly opaque. Thereby the first part of the garment may be opaque.

The first part of the garment may as an example be made of polyester, polyamide, cotton or wool with or without elasthane. It is also possible with a mixture or blend of materials, such as poly-cotton, wool-polyester etc. polyester, although several other types of materials are contemplated. For instance, it may be made of polyamide, cotton or wool instead. The same material can of course also be used in the rest of the garment.

It may have an opacity that is higher than 0.6, with advantage higher than 0.7, preferably higher than 0.8, more preferably higher than 0.9 and most preferably higher than 0.95. The first part of the garment may thus limit at least 60 percent of the ambient light, at least 70 percent of the ambient light, at least 80 percent of the ambient, at least 90 percent of the ambient light or at least 95 percent of the ambient light.

The sensor module holder may provide sealing of the sensor module.

The arrangement may additionally comprise a processing module, a connector for mating with a contact of the processing module and a set of flexible conductors interconnecting the sensor module holder with the connector. In this case the set of flexible conductors may run in the garment from the sensor module holder in the first part of the garment to a second part of the garment, to which the connector is linked or at which the connector is provided.

The arrangement may also comprise a cable having a first end that is joined to the set of flexible conductors in the second part of the garment and a second end that is joined with the connector.

The arrangement may additionally comprise a jointing module in the second part of the garment, which jointing module may seal the connection between the first end of the cable and the set of flexible conductors.

The processing module may provide power for the operation of the sensor module. It is furthermore possible that the processing module comprises a processing unit that provides the power for the operation of the sensor module. The processing unit may comprise a data collecting element that receives sensor data from the at least one body sensor.

The arrangement may also comprise at least one motion sensor that provides motion data and a motion state determining element that analyses the motion data and determines a user motion state based on the motion data.

The at least one motion sensor may be provided in the sensor module and/or in the processing module.

The processing module may further comprise a positioning unit for determining a position of the first user when wearing the garment.

The processing module may also comprise a wireless communication interface for sending data. The data may be sent to a situation handling device or to an application in a commander device.

The processing module may additionally comprise at least one alarm button and the processing unit may send an alarm based on a user actuation of the at least one alarm button. The alarm may be sent by the data collecting element, for instance to the situation handling device or to the application in the commander device.

The processing module may also comprise an impact sensor sensing impacts on the garment. In this case the processing unit may send impact data. The impact data may be sent by the data collecting element, for instance to the situation handling device or to the application in the commander device.

The arrangement may additionally comprise a situation presenting control element that controls presentation of data obtained via the data collecting element to a second user at a second location.

The situation presenting control element may additionally analyse data from the processing module, determine that the first user is alive and in a dangerous situation based on the analysis and send an alarm to an alarm centre if the first user is deemed to be in a dangerous situation.

The arrangement may additionally comprise the situation handling device. The situation handling device may for instance be provided in the cloud. When the arrangement comprises the situation handling device, the situation presenting control element may be a part of this device. As an alternative, the situation presenting control element may be a part of the processing module, for instance as an element of the processing unit.

The motion state determining element may also be a part of the processing module, for instance as an element of the processing unit. Alternatively, it may be a part of the situation handling device.

The arrangement may additionally comprise the application of the commander device, which may be an application for the second user and where the second user may be a commander of the first user. The previously mentioned data is then presented via the application.

It should be emphasized that the term "comprises/comprising" when used in this specification is taken to specify the presence of stated features, integers, steps or components, but does not preclude the presence or addition of one or more other features, integers, steps, components or groups thereof.

### BRIEF DESCRIPTION OF THE DRAWINGS

The present invention will now be described in more detail in relation to the enclosed drawings, in which:
fig. 1 schematically shows a garment with a sensor module holder that holds a sensor module, where the sensor module holder is interconnected with a processing module via a set of flexible conductors and a cable,
fig. 2 shows the sensor module in the sensor module holder being connected to the set of flexible conductors,
fig. 3 shows the sensor module in the sensor module holder and the set of flexible conductors when the sensor module holder is fastened to the garment,
fig. 4 shows an exploded view of the sensor module, sensor module holder and the set of flexible conductors,
fig. 5 shows a jointing module used to interconnect the set of flexible conductors with the cable,
fig. 6 shows the sensor module holder with sensor module being connected to the jointing module via the set of flexible conductors and the processing module being connected to the jointing module via the cable,
fig. 7 schematically shows the processing module communicating with a situation handling device via a mobile network as well as a commander device with an application that also communicates with the situation handling device,
fig. 8 schematically shows a number of sensors in the sensor module and a number of units in the processing module communicating with each other via the set of flexible conductors,
fig. 9 schematically shows one realization of a processor unit in the processing module,
fig. 10 schematically shows one realization of the situation handling device,
fig.11 schematically shows one realization of an arrangement for monitoring a first user at a dangerous first location, which arrangement comprises the situation handling device, application and garment with sensor module holder, sensor module, jointing module, cable, connector and processing module,
fig. 12 shows a flow chart of a number of method steps being performed by the situation handling device, and
fig. 13 schematically shows a view of a screen being displayed by the app in the commander device.

### DETAILED DESCRIPTION OF EMBODIMENTS

In the following description, for purposes of explanation and not limitation, specific details are set forth such as particular architectures, interfaces, techniques, etc. in order to provide a thorough understanding of embodiments described herein. However, it will be apparent to those skilled in the art that the present invention may be practiced in other embodiments that depart from these specific details. In other instances, detailed descriptions of well-known devices, circuits, and methods are omitted so as not to obscure the description with unnecessary detail.

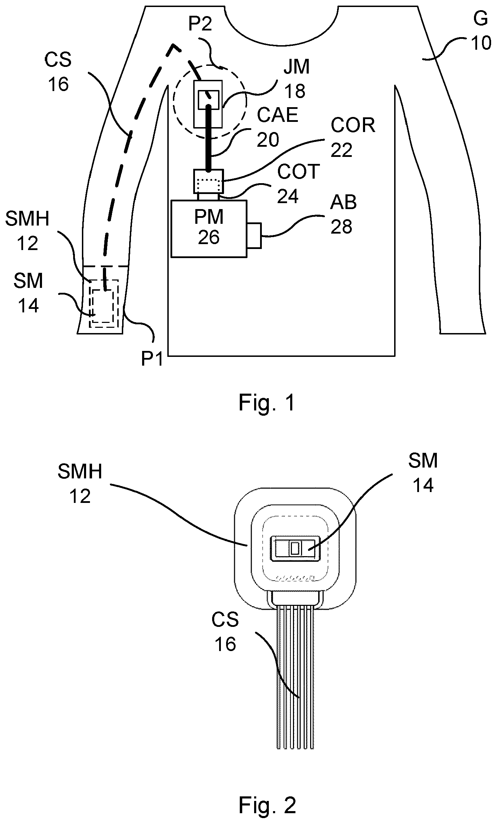

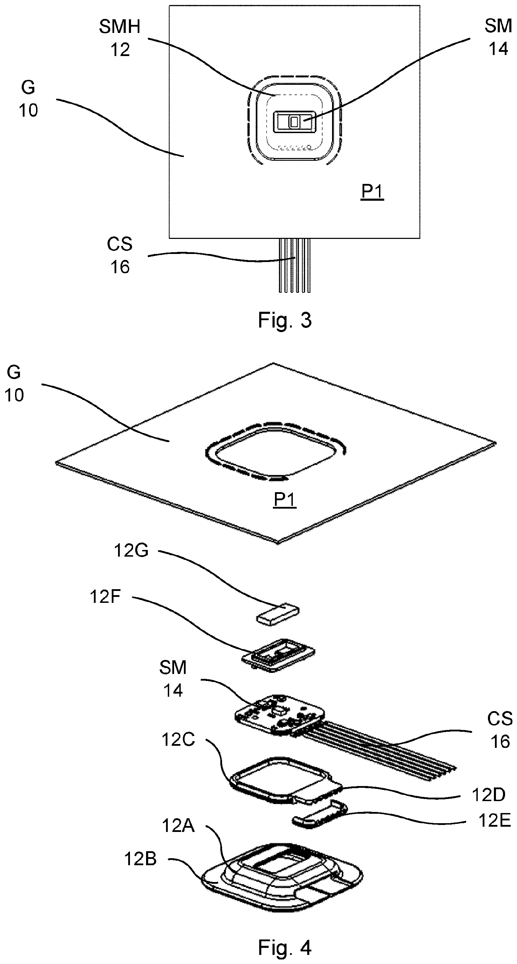

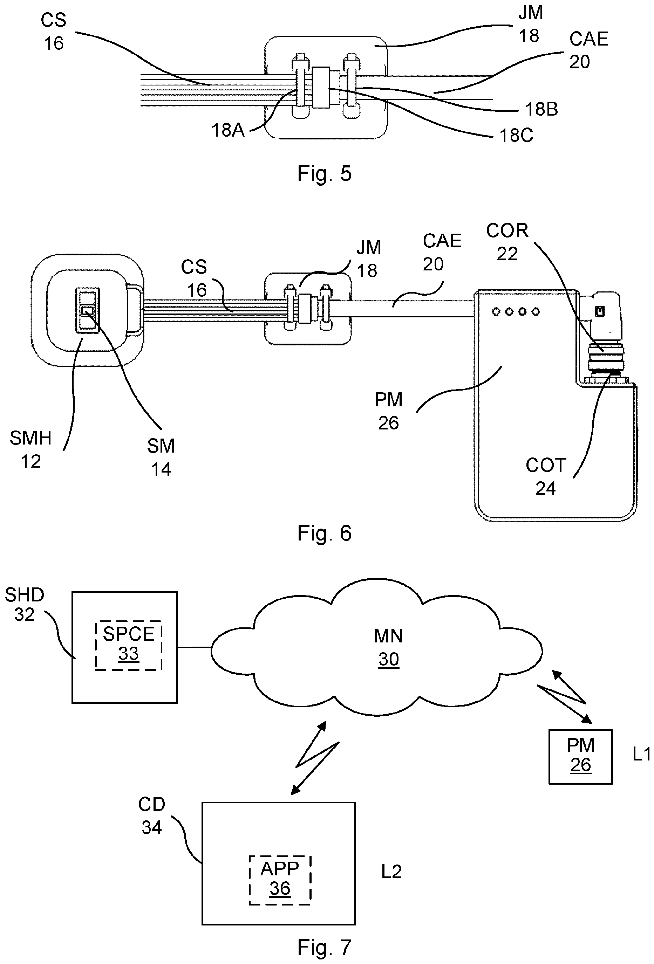

A special garment will now be discussed with reference being made to fig. 1 - 6, where fig. 1 schematically shows the garment with a sensor module holder that holds a sensor module, where the sensor module holder is interconnected with a processing module via a set of flexible conductors and a cable, fig. 2 shows the sensor module in the sensor module holder being connected to the set of flexible conductors, fig. 3 shows the sensor module in the sensor module holder and the set of flexible conductors when the sensor module holder is fastened to the garment, fig. 4 shows an exploded view of the sensor module, sensor module holder and the set of flexible conductors, fig. 5 shows a jointing module used to interconnect the set of flexible conductors with the cable and fig. 6 shows the sensor module holder with sensor module being connected to the jointing module via the set of flexible conductors and the processing module being connected to the jointing module via the cable.

A special garment G 10 is provided for a first user, which first user may be a person that works in emergency services, such as a fireman or a policeman. Thereby the garment 10 may be worn by the first user. The garment 10 may be a garment provided for the upper body of the first user, such as a shirt, a jacket or a sweater. Furthermore, the garment has a first area or first part P1, which in this case is an area around a part of the sleeve of the garment 10. The first part P1 comprises a sensor module holder SMH 12 in which a sensor module SM 14 is to be placed. Thus, the garment 10 comprises a sensor module holder 12 for the sensor module 14, which sensor module holder 12 is located in and fastened to the first part P1 of the garment 10. Thereby, the sensor module holder 12 is located in and fastened to the first part P1 of the garment 10. The sensor module holder 12 may be covered with silicone or epoxy to make it water-proof and avoid corrosion as well as for protecting the sensor module 14. It may additionally be tightly fastened to the garment, for instance through being sewn. The first part P1 is with advantage a part that is to cover a part of the body in which body measurements can be made. The first part P1 may with advantage be the sleeve hem and/or a padding of the garment 10 that is to cover the wrist of the first user. Furthermore, when the first user wears the garment 10, it presses the sensor module holder 12 with the senor module 14 against above-mentioned part of the body of the first user. It may for instance press the sensor module 14 against the wrist of the first user.

The first part P1 of the garment 10 may as an example be made of polyester, although several other types of materials are contemplated. For instance, it may be made of polyamide, cotton or wool instead. The polyester, polyamide, cotton or wool could all be mixed with elasthane. It is also possible with a mixture or blend of materials, such as poly-cotton, wool-polyester etc. The same materials can of course also be used in the rest of the garment.

The first part P1 may have means for holding the sensor module holder with sensor module tight against the body, in this case against the wrist. There are several ways in which this can be done. The first part P1 may be equipped with buttons, which upon buttoning presses the sensor module 14 and sensor module holder 12 against the wrist. It may also have straps that can be used to strap the first part P1 of the garment 10 to the wrist. As yet another alternative the material of the first part P1 may have elastic properties that are used to press at least some of the first part P1 onto the wrist. The material may additionally be at least partly non-transparent or opaque. Therefore, it may be opaque. It may for instance have an opacity that is higher than 0.6, with advantage an opacity that is higher than 0.7, preferably an opacity that is higher than 0.8, more preferably an opacity that is higher than 0.9 and most preferably an opacity that is higher than 0.95. The first part of the garment may thus limit at least 60 percent of the ambient light, at least 70 percent of the ambient light, at least 80 percent of the ambient, at least 90 percent of the ambient light or at least 95 percent of the ambient light.

Thereby the sensor module 14 may be protected against ambient light. There may additionally be a hole for a thumb in the first part P1 and if present this hole may be used to keep the sensor module in the right place.

The sensor module holder 12 may include an interface to a set of flexible conductors CS 16 and when the sensor module 14 is placed in the sensor module holder 12, contacts of the sensor module 14 are connected to the interface. Alternatively, the conductors may be directly connected to the sensor module through soldering.

As is indicated above, the sensor module holder 12 may provide sealing of the sensor module 14 and the set of flexible conductors 16. The sensor module holder may comprise a tray 12C, which is joined to a first conductor sealing part 12D. The sensor module 14 may be placed on the tray 12C and the conductors of the set of flexible conductors 16 placed adjacent the first conductor sealing part 12D and soldered to the sensor module 14. A second conductor sealing part 12E may be placed against the first conductor sealing part 12D, so that the set of flexible conductors are sandwiched between and sealed by the conductor sealing parts 12D, 12E. The sensor module holder 12 may additionally comprise a frame 12A that surrounds and is adapted to the shape of the sensor module tray 12C and first and second conductor sealing parts 12D, 12E. The sensor module holder 12 may also comprise a window holder 12F and a window 12G that are to be placed in an opening of the frame 12A, which opening is aligned with and covers any sensors of the sensor module 14. The frame 12A, tray 12, first and second conductor sealing parts 12D, 12E window holder 12F and window 12G together provide a sealed enclosure in which the sensor module 14 may be placed.

Furthermore, outside of the frame 12A, the sensor module holder 12 may be equipped with a wrapping 12B, which wrapping 12B may act as a flange for the frame 12A.

When placing the sensor module holder 12 with sensor module 14 in the garment 10, the frame 12A of the sensor module holder may be pushed through a hole of the first part P1 of the garment 10. The shape of the frame 12A may be adapted after the shape of the hole so that the sensor module holder (without wrapping) fits tightly in the hole of the garment.

The wrapping 12B, which does not fit in the hole, may then be fastened to this first part P1, for instance through being sewn with stiches.

As an example, the frame and wrapping 12A, 12B may be made of silicone or a soft plastic such as a thermoplastic elastomer (TPE). The tray 12C with first and second conductor sealing parts 12D,12E and window holder 12F may in turn be made of a thermoplastic material such as acrylonitrile butadiene styrene (ABS), polycarbonate (PC) or similar. The window may be made of glass.

The set of flexible conductors 16, which may be insulated and for instance realized as flexfilm, then runs inside the garment 10 from the first part P1 with the sensor module holder 12 to a second area or second part P2 of the garment 10, in which second part P2 there is a provided a jointing module JM 18, which jointing module 18 interconnects the set of flexible conductors 16 with a connector COR 22. Thereby, the connector 22 is also linked to or provided at the second part P2. Furthermore, the set of flexible conductors 16 also interconnect the sensor module holder 12 with the connector 22. The second part P2 can be of the same material as the first part. However, it can also be of a different material. The material that is used can be any of the previously mentioned materials.

In the present example, the jointing module 18 provides an interface 18C between the set of flexible conductors 16 and a first end of a cable CAE 20, in which interface the conductors of the set 16 may be joined with conductors of the cable 20, for instance through soldering. The set of flexible conductors may be fastened in the jointing module 18 with a first fastening element 18A and the first end of the cable may be fastened in the jointing module 18 with a second fastening element 18B.

The jointing module 18 may provide sealing of the interconnection or interface 18C between the set of flexible conductors 16 and the cable 20.

The second end of the cable 20 is provided with a connector COR 22. Thereby the cable 20 has a first end that is joined to the set of flexible conductors 16 in the second part P2 of the garment 10 and a second end that is joined with the connector 22.

As can be seen a processing module PM 26 has a contact COT 24, via which it can be connected to the connector COR 22. Thus, the connector 22 is provided for mating with the contact 24 of the processing module 26.

The processing module be provided inside a casing, which may be made of a metal, such as steel or aluminum. The casing may be watertight, for instance using one or more sealing gaskets of silicone, TPE or similar materials.

The set of flexible conductors 16, which is typically surrounded by insulation, runs inside the garment 10 and here inside the sleeve of the garment 10 up to the second part P2, where it enters the jointing module 18. It may in this case run between two layers of the garment 10.

The jointing module 18 and the cable 20 with the connector 22 are with advantage provided on an external side of the garment 10, such as on the exterior facing away from the body of the first user. The processing module 26 may be placed in a pocket of the garment, such as a breast pocket, or a belt to be worn by the first user. Therefore, the second part can have several locations depending on the location of the processing module 26. Furthermore, the cable 20 is in essence only provided for providing some flexibility and ease of connecting the processing module 26 to the set of flexible conductors 16. Therefore, it is possible that the cable 20 is omitted. The set of flexible conductors 16 may in this case be directly connected to the connector 22 in the jointing module 18. The jointing module 18 and connector 22 may in this case be provided in the previously described pocket. Also the jointing module 18 maybe covered with silicone or epoxy to make it water proof and avoid corrosion. It may additionally be tightly fastened to the garment, for instance through being sewn.

As can be seen in fig. 1, the processing module 26 is also equipped with an optional alarm button AB 28. There may be at least one alarm button on the processing module 26. This will be discussed more later on. Optionally there may be two alarm buttons.

The alarm button 28 may as an example be made of silicone, TPE or similar soft rubbers.

The first user may be at a first location L1. Thereby also the processing module 26 may be located at the first location L1. As can be seen in fig. 7, the processing module 26, when at this first location L1, communicates with a situation handling device SHD 32, which is done via a mobile network MN 30. The situation handling device 32 here also includes a situation presenting control element SPCE 33.

There may also be a commander device CD 34 which may be a mobile phone, such as a smart phone, with an application APP 36, which application 36 also communicates with the situation handling device 32. The commander device 34 may be located at a second location L2, which may be the location of a second user or commander of the first user. The commander device 34 can also be a laptop or a desk top computer that communicates with the mobile network via the Internet.

The situation handling device 32 may be a server connected to the backbone of the mobile network 30. It may also be provided in the cloud, i.e., as functionality in a data centre. However, it may also be a separate server in a local area network connected to the mobile network via the Internet.

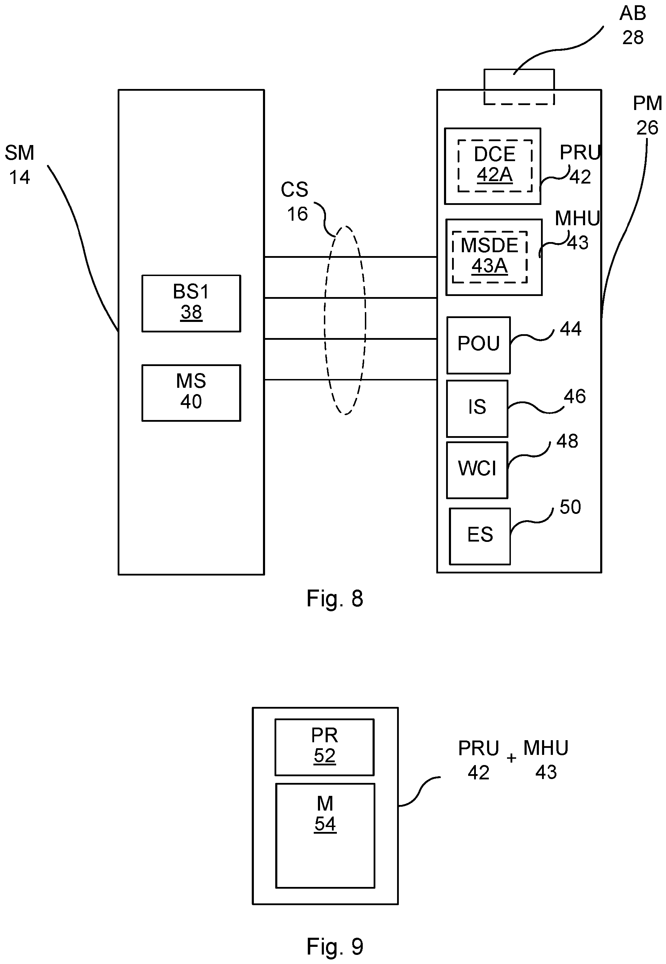

Fig. 8 schematically shows one variation of the sensor module SM 14 being connected to the processing module PM 26 via the set of flexible conductors 16. Here the sensor module holder, jointing module, cable, connector and contact have all been omitted.

The sensor module 14 comprises at least one body sensor. In this case it comprises a first body sensor BS1 38, which may be a pulse or heart rate sensor for sensing heartrate and resting heartrate. It is possible with other types of body sensors such as blood pressure sensors and/or blood oxidization level sensors. Optionally, the sensor module 14 also comprises at least one motion sensor, MS 40, which maybe a gyroscope or an accelerometer that provides motion data.

In addition to the alarm button AB 28, the processing module PM 26 comprises a processing unit PRU 42, a wireless communication interface WCI 48, an energy source ES 50, for instance in the form of a battery, an optional motion handling unit MHU 43,an optional positioning unit POU 44, for instance a Global Positioning (GPS) unit, and an optional impact sensor IS 46, which may also be an accelerometer. Here the processing unit comprises a data collecting element DCE 42A and the motion handling unit 43 comprises a motion state determining element MSDE 43A. The processing module 26 may also comprise a charging port for charging of the energy source 50 (not shown).

Fig. 9 shows one realization of the processing unit PRU 42 optionally together with the motion handling unit MHU 43. The two units 42,43 may be realized as a processor PR 52 with a memory M 54 comprising computer instructions or computer program code for operating on data received from at least one body sensor, at least one motion sensor, the positioning unit 44, impact sensor 46 and alarm button 28. The memory 54 may be a non-transitory storage medium.

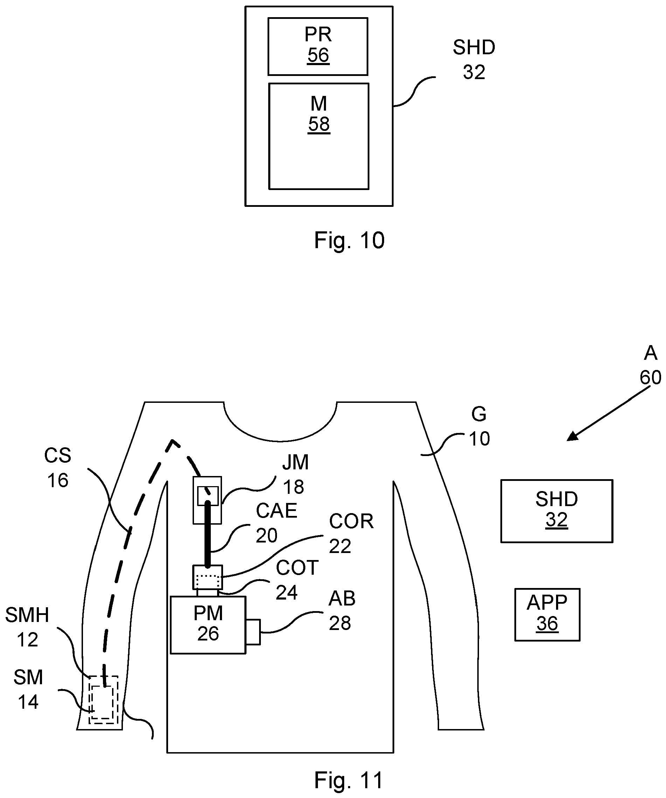

As can be seen in fig. 10, the situation handling device SHD 32 may in a similar manner be realized as a processor PR 56 with a memory M 58 comprising computer instructions or computer program code for operating on the data received from the processing module 26. Also this memory 58 may be a non-transitory storage medium.

As can be seen in fig. 11, the situation handling device SHD 32, application APP 36 and garment G 10 with sensor module holder SMH 12, sensor module SM 14, set of flexible conductors CS 16, jointing module JM 18, cable CAE 20, connector COR 22, contact COT 24 and processing module PM 26 may form an arrangement 60 for monitoring the first user at the first location.

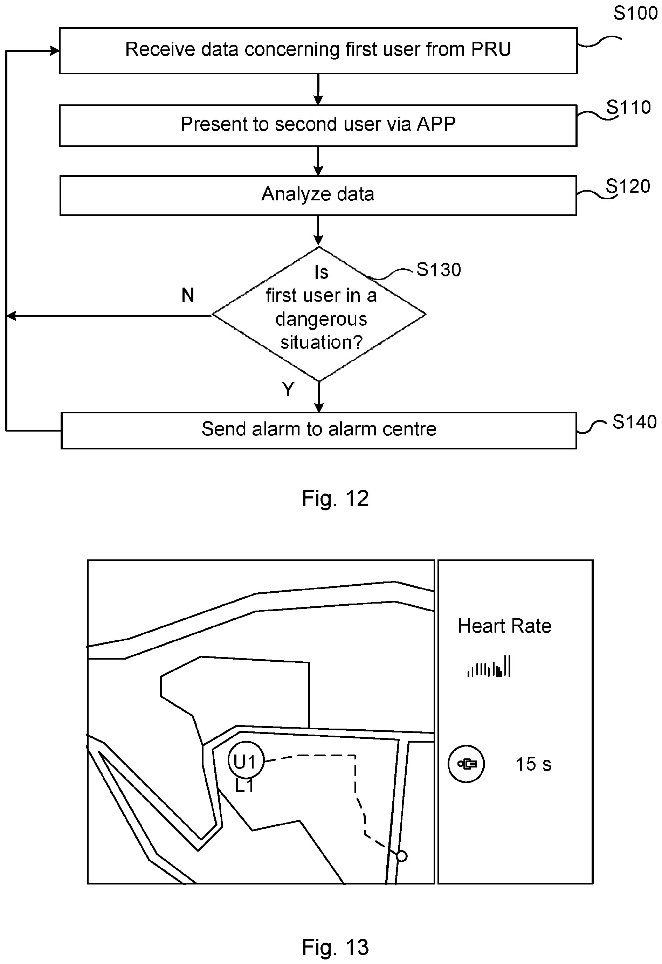

How this arrangement may operate will now be described with reference also being made to fig. 12 and 13, where fig. 12 shows a flow chart of a number of method steps being performed by the situation handling device 32 and fig. 13 schematically shows a view of a screen being displayed by the application 36 in the commander device 34.

The first user may be a firefighter or a policeman and may because of this be visiting dangerous or hazardous locations. The garment 10 may be provided for protecting the first user. Furthermore, because the environment may be hazardous, it may also be of interest to monitor the health of the first user. For this reason, the garment 10 is equipped with the sensor module 14. It may be of interest to place the sensor module 14 in the garment 10 because then the movements and activities of the first user are not impeded by the sensor module 14. At the same time the sensor module 14 may be placed at a part of the body where measurements can easily be made. The sensor module 14 may also need to be small, which means that it should not have any power or energy sources.

At the same time, it is important that the sensor measurements can be made as correctly and efficiently as possible. A sensor is often realized using a LED and such a LED may need to be pressed onto the body in order to correctly measure a property of the body such as blood pressure. Furthermore, such a sensor is often sensitive to ambient light. Therefore, the ambient light levels may also need to be kept low.

The sensor module holder 12 holds the sensor module 14 at the intended position in the garment 10. The sensor module 14 can also be slid into the sensor module holder 12, which simplifies assembly while also allowing skin contact to be made without any added wearables. The sensor module holder 12 also protects the sensor module 14. A wall of the sensor module holder 12 that faces the body may be transparent. Thereby the sensor module holder 12 protects the sensor module 14, while at the same time enabling quality measurements to be made. The first part P1 of the garment 10 may additionally be used to press the body sensors 38 against the part of the body corresponding to the first part P1 of the garment 10. The material may also completely cover the sensor module holder 12 and ensure that the amount of ambient light that reaches the body sensors of the sensor module 14 is limited, and therefore the body data is of good quality. Furthermore, through providing a separate processing module 26, it is possible to remotely power the sensor module 14. Thereby, the sensor module can be kept small and lightweight.

The processing module 26, which is heavier and bulkier than the sensor module 14, can on the other hand be held at a part where the influence on the first user is minimal. It does therefore not impede the movement of the first user. The processing module 26 is powered by the energy source 50. The processing module 26 can also use power from the energy source for powering the sensor module 14. It is possible that the process control unit controls the provision of this power for the operation of the sensor module 14.

Additionally, the processing unit 42 can be used to provide additional processing that does not require closeness to the part of the body where measurements are being made. It can also include additional units, such as the positioning unit 44, which determines the position of the first user when wearing the garment 10, and the impact sensor 46, which senses impacts on the garment 10. The impact sensor may be a high g-force 200G accelerometer that detects severe impacts such as bullets or falling trees. Furthermore, the set of flexible conductors 16 allows the signals from the sensor module 14 to be transferred to the processing module 26 without negatively influencing the movement of the first user. He or she may be allowed to move freely.

The jointing module 18 on the other hand interconnects the cable 20 with the set of flexible conductors in a secure way. As it may be tightly fastened to the second part P2 of the garment 10, it can also act as an anchor point. In this case, it bridges the harsh environment outside the garment with the moist environment inside.

The motion state determining element 43A of the motion handling unit 43 receives motion data from the motion sensor 40. It then analyses the motion data and determines a user motion state based on the motion data. Thereby, the motion state determining element 43A of the motion handling unit 43 determines one or more user motion states based on the motion or movement data.

One state may be that the first user is lying down, another state may be that the first user is standing up and yet another state may be that the first user is standing up and moving, such as walking or running. Another state may be that the first user is on the ground and creeping or crawling. Other states may be that the first user is moving up and down. The motion state determining element 43 may for instance have been trained using various motion data to be able to detect various user motion states. The motion state determining element may thus have learnt the states through machine-learning. The determined states may be provided from the motion state determining element 43A to the data collecting element 42A.

The data collecting element 42A of the processing unit 42 also receives body sensor data from the at least one first body sensor 38. It may also receive position data about the position of the first user from the positioning unit 44, impact data about impacts on the user from the impact sensor 46 and data about user actuation of the alarm button 28.

The data collecting element 42A of the processing unit 42 may furthermore forward data to the situation presenting control element 33 in the situation handling device 32. It may for instance forward a user movement state that has been determined for the first user together with body data from the body of the first user. This data may be time stamped. It may also forward position data about the position of the first user, impact data from the impact sensor and an alarm signal if the alarm button is being pressed or actuated by the first user. Also this data may be time stamped. All the data may then be sent or transmitted to the situation handling device 32 using the wireless communication interface 48 and mobile network 30, where an alarm may be sent based on a user actuation of the alarm button 28. In some variations the data may be directly sent to the application 36 in the commander device 34 instead.

The situation presenting control element 33 of the situation handling device 32 may operate in a loop. In this loop, it may first receive data concerning the first user from the data collecting element 42A of the processing unit 42, S100. The situation presenting control element 33 of the situation handling device 32 then goes on and controls the presentation of the data obtained from the data collecting element 42A to the second user via the application 36 of the commander device 34, S110. It may for instance make the application 36 present the body data, the determined motion state and how long the first user has had the determined motion state. The time that the user has had the state may be determined based on the time stamps of the motion states. The situation presenting control element 33 may also make the application 36 display the current position of the first user at the first location L1 as well as the route the first user has travelled to this current position from a starting point. In an example shown in fig. 13, the heart rate of the first user U1 is displayed with the motion state lying down, where the first user U1 has had this state for 15 seconds. Moreover, the movement of the first user U1 to the current position at the first location L1 from an originating position is also shown on a map.

The situation presenting control element 33 may also make the application display impact data from the impact sensor. If the first user has pressed the alarm button, this may also be signaled to the second user.

The second user may then take an action based on the displayed data. If for instance the first user U1 has pressed the alarm button 28, the second user may determine that an emergency situation is at hand. He or she may then order a rescue team to be sent out to the first user U1. This may also be done if the impact data indicates that the first user U1 has been hit hard, the body data indicates that the first user U1 has a high pulse and the motion state indicates that the first user U1 is lying down. When this is done, the body data and the position may automatically be included in such an order.

On the other hand, if the first user U1 has no pulse and has been lying down for some time then it is possible that no emergency activity is needed, because the first user has unfortunately died.

Optionally the situation presenting control element 33 of the situation handling device 32 may also analyze the data received from the processing module 26, S120, and perform an activity based on the analysis. It may for instance determine that the first user U1 is alive and in a dangerous situation in the first case, S130, and send an alarm to an alarm center if the first user U1 is deemed to be in a dangerous situation, S140, where the alarm may also include the position and body data of the first user U1. It is also possible that the first user U1 is moving about and has a normal pulse, in which case the first user U1 is not in a dangerous situation, S130, and no alarm is being sent.

The situation presenting control element 33 then returns to the beginning of the loop.

It can in this way be seen that the second user is informed about the first user and that optionally also an alarm is automatically generated if the first user is deemed to be in a dangerous situation.

Thereby the safety of the first user may be increased.

The situation handling device can perform the same type of operation for a number of users. There may thus be a first group of users, comprising the first user, about which the situation handling device presents information to the second user via the application 36.

There are several ways in which the invention may be varied. The garment may be a garment provided for the lower body of the first user, such as a pair of trousers. As was mentioned above it is possible that the sensor module includes more sensors. It is also possible to omit the motion sensor from the sensor module. The processing module may likewise include more sensors, for instance a temperature sensor and/or a motion sensor. Such a motion sensor may for instance be a part of the motion handling unit. This motion sensor can with advantage be used together with the motion state determining element instead of or in addition to the previously-described motion sensor in the sensor module. It is also possible with fewer sensors. It is for instance possible that the impact sensor and the position sensor are omitted. There may thus be no sensors at all in the processing module. In fact in some embodiments the processing module may be omitted completely. In this case it is possible to omit also the set of flexible conductors, the cable and the jointing unit.

However, if this is done, the sensor module may need to include an energy source.

From this it can also be understood that there may be variations in the arrangement. The arrangement may in its simplest form only comprise the garment, sensor holder module and sensor module. If the arrangement comprises the processing module, then the set of flexible conductors are needed as well as perhaps also the jointing module.

Furthermore, if there is a processing module then this would need to at least include the processing unit with the data collecting element. It may with advantage also include the wireless communication interface. A processing module without wireless communication interface may have a non-volatile memory for storing of data concerning the first user as may an arrangement only comprising the garment, sensor module holder and sensor module. If the processing module comprises the wireless communication interface, it may be used for communicating with the situation handling device or directly with the application. In this case the situation handling device and/or the application may also be included in the arrangement. As can be seen above, the situation presentation control element, if present, could be provided in the processing module or in the situation handling device. If the sensor module comprises the motion sensor, then the arrangement also comprises the motion state determining element, which may be provided in the processing module or in the situation handling device.

While the invention has been described in connection with what is presently considered to be most practical and preferred embodiments, it is to be understood that the invention is not to be limited to the disclosed embodiments, but on the contrary, is intended to cover various modifications and equivalent arrangements. Therefore, the present invention is only to be limited by the following claims.

## Claims

1. An arrangement (60) for monitoring a first user (U1) at a first location (L1), the arrangement (60) comprising:
a sensor module (14) including at least one body sensor (38), and
a garment (10) to be worn by the first user (U1), the garment comprising a sensor module holder (12) for the sensor module (14), the sensor module holder (12) being located in and fastened to a first part (P1) of the garment (10) that is to press the sensor module holder (12) with the sensor module (14) against a part of a body of the first user (U1) when the first user (U1) wears the garment (10).

2. The arrangement (60) according to claim 1, wherein the first part (P1) of the garment (10) is at least partly opaque.

3. The arrangement (60) according to claim 1 or 2, wherein the sensor module holder (12) provides sealing of the sensor module (14).

4. The arrangement (60) according to any of claims 1 - 3, further comprising a processing module (26), a connector (22) for mating with a contact (24) of the processing module (26) and a set of flexible conductors (16) interconnecting the sensor module holder (12) with the connector (22), the set of flexible conductors (16) running in the garment (10) from the sensor module holder (12) in the first part (P1) of the garment to a second part (P2) of the garment, to which the connector (22) is linked.

5. The arrangement (60) according to claim 4, further comprising a cable (20) having a first end that is joined to the set of flexible conductors (16) in the second part (P2) of the garment (10) and a second end that is joined with the connector (22).

6. The arrangement (60) according to claim 5, further comprising a jointing module (18) in the second part (P2) of the garment (10), which jointing module (18) seals the connection between the first end of the cable (20) and the set of flexible conductors (16).

7. The arrangement according to any of claims 4 - 6, wherein the processing module (26) is operative to provide power for the operation of the sensor module (14) and comprises a data collecting element (42A) operative to receive sensor data from the at least one body sensor (38).

8. The arrangement (60) according to claim 7, further comprising at least one motion sensor (40) that provides motion data and a motion state determining element (43A) being operative to analyse the motion data and determine a user motion state based on the motion data.

9. The arrangement (60) according to any of claims 4 - 8, the processing module (26) further comprising a positioning unit (44) for determining a position of the first user when wearing the garment (10).

10. The arrangement (60) according to any of claims 4 - 9, the processing module (26) further comprising a wireless communication interface (48) for sending data.

11. The arrangement (60) according to claim 10, the processing module (26) further comprising at least one alarm button (28) and the processing unit (42) being further operative to send an alarm based on a user actuation of the at least one alarm button (28).

12. The arrangement (60) according to claim 10 or 11, the processing module (26) further comprising an impact sensor (46) sensing impacts on the garment (10).

13. The arrangement (60) according to any of claims 10 - 12, further comprising a situation presenting control element (33) being operative to control presentation of data obtained via the data collecting element (42A) to a second user at a second location (L2).

14. The arrangement (60) according to claim 13, further comprising an application (36) for the second user, via which said data is presented.

15. The arrangement (60) according to claim 13 or 14, the situation presenting control element (33) being further operative to analyse data from the processing module (26), determine that the first user (U1) is alive and in a dangerous situation based on the analysis and send an alarm to an alarm centre if the first user (U1) is deemed to be in a dangerous situation.
